(19) Europäisches Patentamt
European Patent Office
Office européen des brevets

(11) Numéro de publication : **0 312 423 B1**

# (12) FASCICULE DE BREVET EUROPEEN

(45) Date de publication du fascicule du brevet : **11.03.92 Bulletin 92/11**

(51) Int. Cl.$^5$ : **A61K 31/19**, A61K 35/74, C12P 13/02

(21) Numéro de dépôt : **88402480.3**

(22) Date de dépôt : **30.09.88**

## (54) Agents anti-parasitaires, procédé d'obtention de ces produits et compositions anti-parasitaires, et notamment antimalariques, contenant lesdits produits.

(30) Priorité : **02.10.87 FR 8713623**

(43) Date de publication de la demande : **19.04.89 Bulletin 89/16**

(45) Mention de la délivrance du brevet : **11.03.92 Bulletin 92/11**

(84) Etats contractants désignés : **AT BE CH DE ES FR GB GR IT LI LU NL SE**

(56) Documents cités :
**MOL.PHARMACOL., vol. 30, no. 4, p. 364-369, L.W.SCHEIBEL et al.:"Antimalarial activity of selcted aromatic chelators. IV. Cation uptake by Plasmodium falciparum in the presence of oxines and siderochromes"**
**JOURNAL OF MEDICINAL CHEMISTRY, vol. 15, no. 11, 1972, p. 1194-1195; J.B.HYNES et al.:"Hydrocyclamine derivatives as potential antimalarial agents. 2. Hydroxamates and amidoximes"**
**ANN.PHARMACEUTIQUES FRANCAISES, vol. 44, no. 5, 1986, p. 329-338; Masson, Paris, FR. A.FAVIER et al.:"Les sidérophores II. Leur role biologique et leur intéret en pathologie et en thérapeutique humaine"**

(56) Documents cités :
**ANN.PHARMACEUTIQUES FRANCAISE, vol. 44, no. 4, 1986, pages 255-268 Masson, Paris FR, J.-L. PIERRE eet al.:"Les sidérophores. I. Etudes structurales et physicochimiques"**
**INFECTION AND IMMUNITIY, vol. 54, no. 3, décembre 1986, p. 603-608; Am.Soc. for Microbiology, US X.NASSIF et al.: "Correlation of the virulence of Klebsiella pneumoniae K1 and K2 with the presence of a plasmid encoding aerobactin"**
**DISSERTATION ABSTRACTS INTERNATIONAL, vol. 46, no. 2, August 1985, page 505B, colonne de droite D.V.L. APPANNA: "Biosynthesis of aerobactin"**

(73) Titulaire : **INSTITUT PASTEUR**
**28, rue du Docteur Roux**
**F-75724 Paris Cédex 15 (FR)**

(72) Inventeur : **Gysin, Jurg**
**Institut Pasteur**
**Cayenne (GY)**
Inventeur : **Crenn, Yves**
**Institut Pasteur**
**Cayenne (GY)**
Inventeur : **Pereira da Silva, Luiz**
**4 Square Port Royal**
**F-75013 Paris (FR)**
Inventeur : **Breton née Braun, Catherine**
**9 Place de Séoul**
**F-75014 Paris (FR)**

(74) Mandataire : **Ores, Irène et al**
**CABINET ORES 6, Avenue de Messine**
**F-75008 Paris (FR)**

EP 0 312 423 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à des produits sidérophores bactériens inhibiteurs de parasites endogènes, à des procédés d'obtention de ces produits, à des compositions anti-parasitaires et notamment anti-malariques contenant lesdits produits.

Dans la littérature, il est spécifié que le fer est un élément essentiel au développement microbien ; cependant, bien qu'élément largement répandu dans la nature, le fer sous forme ferrique est très peu soluble et est donc peu disponible directement sans le concours de mécanismes ayant une haute affinité pour ces formes ferriques et qui permettent leur passage à travers la paroi bactérienne, afin d'assurer la multiplication des bactéries, surtout lorsque le milieu est pauvre en fer.

Les entérobactéries sécrètent un chélateur de fer, l'entérocheline à structure phénolique; certaines souches d'Escherichia coli, Aerobacter aerogenes, Salmonella sp. et Klebsiella pneumoniae entre autres sécrètent en plus un chélateur du fer non phénolique appelé aérobactine.

Dans l'article de WARNER et al. (Inf. Immun., 1981, 33, 2, p. 540-545), il est précisé que E. coli synthétise de l'aérobactine, dont les déterminants génétiques sont situés sur des plasmides appelés ColV plasmide, car identifiés comme étant capables de promouvoir la synthèse de Colicine V.

Dans l'article de NASSIF (Inf. Immun., 1986, 54, 3, p. 603-608), il est montré qu'un des facteurs de la virulence de certaines souches de Klebsiella pneumoniae est la production d'aérobactine par lesdites souches.

Dans l'article de SCHEIBEL et call. (Mol. Pharmacol. 1986, 30(4), p. 364-369), il est montré que certains complexes de fer, renfermant par exemple de la mycobactine P, ont une activité antimalariale mais ne pénètrent pas dans les cellules injectées.

De plus, seules les souches aptes à produire de l'aérobactine peuvent continuer à se multiplier dans un milieu pauvre en fer.

La production d'aérobactine est liée à la présence d'un plasmide.

Dans l'article de WILLIAMS et al. (Inf. Immun., 1986, 51, 3, p. 942-947), il est précisé que, bien que l'aérobactine ait une affinité moindre pour les dérivés ferriques que l'entérocheline, elle possède des propriétés physiologiques et de régulation qui permettent un apport en fer, pour la croissance bactérienne, en quantités plus importantes que l'entérocheline; en effet, l'aérobactine stimule la croissance bactérienne à des concentrations 500 fois plus faibles que celles de l'entérocheline.

Par ailleurs, CLARK et al. (Nature, 1976, 259, 309-311 et Parasitol., 1977, 74, 9-18) ont montré que certaines espèces bactériennes sont reconnues comme ayant des propriétés immunostimulantes et sont utilisées pour protéger les souris contre le parasite murin Babesia spp. et contre Plasmodium spp.

Certains composés présentant une action anti-parasitaire sont décrits dans l'Art antérieur.

En particulier :

La quinine, qui agit par fixation sur l'ADN des schizontes, essentiellement sur les formes jeunes et provoque la destruction de ceux-ci en 2 à 4 jours; toutefois celle-ci présente une certaine toxicité.

Les dérivés de la 4-aminoquinoléine, notamment la chloroquine, qui agissent par inhibition de la polymérase de l'ADN des parasites, empêchant sa réplication et provoquent la destruction de ceux-ci en 2 à 4 jours. Ces dérivés ne présentent pas une toxicité importante; cependant de plus en plus de souches, notamment de Plasmodium falciparum, sont résistantes à ces dérivés.

La présente invention s'est fixé pour but de pourvoir à une nouvelle famille d'anti-parasitaires d'action spécifique sur les parasites endocellulaires, notamment contenus dans les globules rouges humains, qui se distinguent des anti-parasitaires proposés dans l'Art antérieur en ce qu'ils agissent selon un mécanisme d'action totalement différent et original dans ce domaine, à savoir une action de chélateur du fer, entraînant une privation en fer du parasite, associée à une action de toxine, ce qui a pour effet de permettre des traitements d'efficacité plus rapide, à des doses moindres qu'avec les composés de l'Art antérieur, les anti-parasitaires conformes à l'invention présentant de plus une faible toxicité.

La présente invention a pour objet l'utilisation d'une substance constitué par un produit sidérophore comprenant au moins un groupe hydroxamate, au moins une liaison peptidique, et un poids moléculaire inférieur à 1000 D, et présentant la propriété de pénétrer dans les cellules, notamment dans les globules rouges, pour l'obtention d'un médicament destiné à une utilisation anti-parasitaire, notamment antimalarique, spécifique et non réversée par le fer, vis-à-vis des parasites endocellulaires.

Conformément à l'invention, ladite substance est avantageusement de l'aérobactine de formule I ci-après :

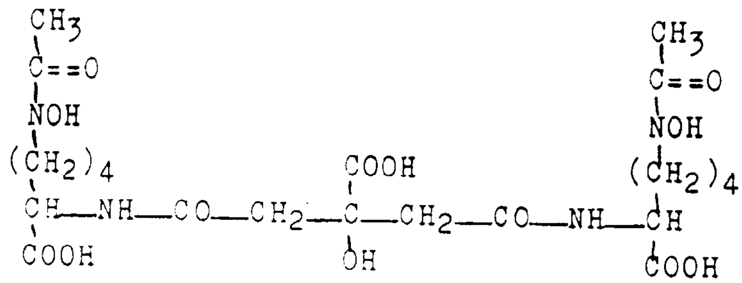

(I)

laquelle formule englobe également les produits dérivés par substitution.

Les produits de formule I inhibent la multiplication des parasties endocellulaires par une action combinée de chélateur de fer et de toxine.

Lesdits composés selon l'invention sont non toxiques pour l'homme ou l'animal.

Un procédé d'obtention avantageux de l'aérobactine par exemple, comprend la culture dans un milieu dépourvu de fer, d'une entérobactérie pathogène, notamment Klebsiella pneumoniae K5a 1101, puis la purification de l'aérobactine obtenue, à partir du surnageant de culture.

La purification comprend l'introduction du surnageant de culture dans une colonne de chromatographie échangeuse d'ions puis une élution.

La portion d'éluat contenant notamment l'aérobactine subit une filtration sur gel Sephadex® G50.

Les composants actifs selon l'invention peuvent être formulés en compositions anti-parasitaires.

Selon un autre aspect de la présente invention, on fournit des compositions pharmaceutiques comprenant une quantité active d'au moins une substance anti-parasitaire conforme à l'invention, ayant une activité plasmocidine tant chez l'homme que chez l'animal, éventuellement associé à un véhicule d'administration approprié.

Les compositions pharmaceutiques peuvent être administrées notamment par voie orale, parentérale. Lorsqu'elles sont administrées par voie orale, lesdites compositions peuvent être présentées sous forme retard notamment par adsorption du/des principe(s) actif(s) sur du phosphate de calcium.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de préparation des produits selon l'invention par voie de synthèse, de mise en évidence de l'activité anti-parasitaire desdits produits conformes à l'invention.

Il doit être bien entendu, toutefois, que ces exemples sont donnés uniquement à titre d'illustration de l'objet de l'invention, dont ils ne constituent en aucune manière une limitation.

## EXEMPLE

### EXEMPLE 1

- Tests de caractérisation de l'activité anti-parasitaire des produits conformes à la présente invention, dite activité plasmocidine.

1. Mise en évidence de l'inhibition de la multiplication des parasites

- In vitro, cette inhibition est associée à la sécrétion par les bactéries d'une substance de PM = 300, chélatrice du fer, un hydroxamate, appelé aérobactine. L'activité "plasmocidine" a été observée en fonction du milieu de culture bactérienne (Tableau I) et de la concentration finale (exprimée en %), en aérobactine, calculée à partir du titrage.en hydroxamate du surnageant de bactéries, dans le milieu de culture des parasites (Tableau II). Les surnageants de culture de Klebsiella pneumoniae K5a ont été introduits dans des cultures de Plasmodium falciparum comme décrit dans le Tableau I. Le pourcentage de schizontes tués est estimé comme indiqué dans la figure 1. Cette inhibition n'a pu être mise en évidence que lorsque les bactéries sont mises dans des milieux de cultures dépourvus de fer tel que M9 ou RPMI.

Le tableau I montre l'activité "plasmocidine" de l'aérobactine en fonction du milieu de culture (déprivé ou non en fer).

TABLEAU I

| Milieu de culture | Croissance bactérienne | Taux d'augmentation de la parasitémie Jour 1 | Jour 3 |
|---|---|---|---|
| L broth (1) | - | 3 à 5 | 10 à 20 |
| | + | 3 à 5 | 10 à 20 |
| RPMI (2) | - | 3 à 5 | 10 à 20 |
| | + | 1 | 1 |
| RPMI* (3) | - | 3 à 5 | 10 à 20 |
| | + | 0,02 | 0,02 |
| RPMI* + Fe | - | 3 à 5 | 10 à 20 |
| | + | 3 à 5 | 10 à 20 |
| M9 (4) | - | 3 à 5 | 10 à 20 |
| | + | 0,1 | 0,02 |
| M9 + Fe | - | 3 à 5 | 10 à 20 |
| | + | 3 à 5 | 10 à 20 |

(1) et (4) sont décrits dans Nassif et Al. (Infect. Immun., 1986, 54, 603-608).

(2) est liquide (GIBCO) et supplémenté en glutamine.

(3) est un milieu RPMI modifié pour permettre la croissance de P. falciparum sans addition de sérum humain.

Klebsiella pneumoniae K5a 1101 est mis en culture dans différents milieux comme indiqué dans le tableau ci-dessus et récolté à $A_{526nm}$ = 1.

Le surnageant de culture est filtré (filtres Millex 0,22 µm) et introduit dans le milieu de culture d'une souche de Plasmodium falciparum FUP; les parasites étant cultivés selon TRAGER et al. (Science, 1976, 193, 673-675) et synchronisés selon la technique développée par LAMBROS et al. (J. Parasitol., 1979, 65, 418-420).

La parasitémie initiale était de 1 à 2% et les parasites étaient en début de phase schizonte. Les surnageants "+" sont les surnageants de culture de Klebsiella pneumoniae; les surnageants "-" sont les surnageants de contrôle (sans croissance bactérienne).

Les surnageants sont introduits dans le milieu à une concentration finale de 10% en aérobactine, concentration calculée à partir du titrage en hydroxamate. La parasitémie a été suivie pendant 3 jours après l'addition des surnageants et estimée par comptage d'au moins 5000 globules rouges sur des frottis colorés au Giemsa.

"+ Fe" signifie que 100 µM de $FeCl_3$ ont été ajoutées dans la culture de parasites en même temps que le surnageant.

Les résultats correspondent à une valeur moyenne de trois expérimentations indépendantes.

Le Tableau II précise l'activité "plasmocidine" de l'aérobactine en fonction de sa concentration finale calculée à partir du titrage en hydroxamate du surnageant de culture.

TABLEAU II

| Surnageant | Concentration finale | Taux d'augmentation de la parasitémie Jour 1 (anneaux) | Jour 2 (schizontes) | Jour 3 (anneaux) |
|---|---|---|---|---|
| K.pneumoniae / RPMI* | 10% | 0,02 | 0,02 | 0,02 |
| | 5% | 0,1 | 0,1 | 0,02 |
| | 2,5% | 1 | 1 | 1 |
| | 1,25% | 2 à 3 | 2 à 3 | 5 à 8 |
| RPMI* | 1,25% - 10% | 3 à 5 | 3 à 5 | 10 à 20 |

L'expérimentation a eu lieu dans les mêmes conditions que celles décrites dans le Tableau I.

Le surnageant de culture de Klebsiella pneumoniae K5a 1101 développée dans un milieu RPMI a été introduit à différentes concentrations en aérobactine (calculées à partir du titrage en hydroxamate) dans les cultures de Plasmodium falciparum. Les résultats correspondent à deux expérimentations indépendantes.

Les résultats présentés dans le Tableau II conduisent à établir une courbe dose/réponse présentée dans la figure 1 ; celle-ci comporte en abscisse la concentration finale en aérobactine, calculée à partir du titrage en hydroxamate du surnageant de culture de K. pneumaniae, exprimée en % et en ordonnée le pourcentage de schizontes tués.

Cette courbe montre l'activité plasmocidine de l'aérobactine.

L'activité du surnageant a été évaluée en calculant le taux d'augmentation de la parasitémie au cours de chaque cycle parasitaire.

Cette courbe est en accord avec les effets observés après une injection intraveineuse d'un surnageant de culture de Klebsiella pneumoniae à des singes infectés par Plasmodium falciparum.

In vitro, le surnageant de culture de Klebsiella pneumoniae K5a inhibe donc la multiplication de Plasmodium falciparum en culture dans des globules rouges humains.

On observe une dégénérescence des schizontes.

- In vivo, des singes Saïmiri sciureus infectés par Klebsiella pneumoniae K5a montrent une certaine résistance à l'infection par Plasmodium falciparum. Cette "résistance" a pu être associée à la sécrétion par les bactéries d'une ou plusieurs substance(s) de poids moléculaire 1000. En effet, si on injecte par voie péritonéale du surnageant de culture de la souche K5a filtré et déplété des substances de PM > 1000, à des singes parasités par Plasmodium falciparum, on observe une inhibition de la multiplication des parasites (figure 2).

Les Auteurs de l'invention ont également observé que Klebsiella pneumoniae protégeait partiellement les sagouins contre les infections à Plasmodium falciparum.

La figure 2 présente en abscisse la durée de l'infection en jours et en ordonnée la parasitémie exprimée en pourcentage.

Quatre singes sont infectés avec $10^8$ parasites (jour 0).

L'aérobactine est partiellement purifiée à partir d'un surnageant de culture de Klebsiella pneumoniae K5a au moyen de filtrations successives sur des membranes AMICON® PM30, AMICON® PM10 et UM2.

La fraction non retenues par les membranes et dont le poids moléculaire est inférieur à 1000 est utilisée.

Les singes Nos. 1 et 2 sont des contrôles.

Les singes Nos. 3 et 4 ont été infectés comme décrit ci-dessus.

Les injections péritonéales d'aérobactine commencent au jour J=4.

Les doses injectées sont exprimées en ml/jour et précisées dans le Tableau III ci-après.

TABLEAU III

| SINGES | DOSES (en ml/j) | | | | | |
|---|---|---|---|---|---|---|
| | J4 | J5 | J6 | J7 | J8 | J9 |
| Singe n° 3 | - | - | 2 | 2 | 2 | 2 |
| Singe n° 4 | 8 | 8 | 8 | 8 | 8 | - |

La parasitémie est mesurée chaque jour, deux heures avant l'injection, par comptage d'au moins 5000 globules rouges sur des frottis sanguins colorés au Giemsa.

2. Identification de la Plasmocidine

L'activité "plasmocidine" a été étudiée à partir de souches de Klebsiella pneumoniae différant par leur sérotype ou leur virulence (Tableau IV).

TABLEAU IV

| Souches bactériennes | Serotype | Virulence | Milieu de croissance | Concentration finale | % schizontes tués | $A_{526nm}$ |
|---|---|---|---|---|---|---|
| 1101 | K5 | + | RPMI* | 20% | 99% | |
| | | | | 10% | 99% | 0.15 |
| et | | | | 5% | 95% à 98% | à |
| | | | | 2,5% | 60% à 70% | 0.20 |
| 1102 | | | | | | |
| | | | RPMI*+ Fe | 2,5% à 20% | 0 | 0.01 |
| 7825 | K1 | + | RPMI* | 20% | 95% à 99% | |
| | | | | 10% | 65% à 75% | 0.10 |
| | | | | 5% | 50% à 60% | à |
| | | | | 2,5% | 30% à 40% | 0.15 |
| | | | RPMI*+ Fe | 2,5% à 20% | 0 | 0.005 |
| 52144 | K1 | - | RPMI* | 2,5% à 20% | 0 | 0.005 |
| | | | RPMI*+ Fe | 2,5% à 20% | 0 | 0.005 |

Les souches K5a 1101 et 1102 ont été isolées des singes Saïmiri sciureus infectés; en ce qui concerne les souches de K. pneumoniae 7825 et 52144, voir NASSIF (Inf. Immun., 1986, 54, 3, p. 603-608). Les nomenclatures des sérotypes correspondent à celles de RICHARD C. [Bull. INST. PASTEUR (Paris), 1982, 80, 127-145].

Les souches virulentes K5a 1101 et K1 7825 ont une activité "plasmocidine", tandis que la souche non virulente K1 52144 n'en a pas.

Il a été montré que la non virulence de la souche K1 52144 est liée à la non production d'aérobactine (NASSIF X., SANSONETTI P.J., Infect., Immunol., 1986, 54, 603-608).

La production d'hydroxamate a été mesurée sur un échantillon de 200 µl de surnageant, comme décrit dans CSAKY (Acta. Chem. Scand., 1948, 2, 450-454).

La toxicité pour Plasmodium falciparum est associée au pic de purification de l'aérobactine obtenu par colonne échangeuse d'ions (Dowex®) et filtration sur gel Sephadex® G50 (cf. figure 3).

La figure 3 montre que l'activité "plasmocidine" est retrouvée après purification de l'aérobactine.

Les résultats suggèrent que l'activité "plasmocidine" est une propriété de l'aérobactine.

L'aérobactine a été purifiée à partir d'un surnageant de culture de Klebsiella pneumoniae K5a 1101 cultivée dans un milieu RPMI*.

Figure 3a : un surnageant de culture (500 ml à pH 7,0) est introduit dans une colonne Dowex®-1 (25 x 2 cm) et élué avec 500 ml d'un gradient de chlorure d'ammonium variant de 0,4 à 1,0 M, à un débit de 30 ml/h.

Les fractions (2,5 ml) sont collectées et l'hydroxamate est dosé selon la méthode de CSAKY.

Figure 3b : . le pool d'hydroxamate (10 à 15 ml) collecté par la chromatographie par échange d'ions est introduit dans une colonne Sephadex® G50 (25 x 2 cm) et élué avec 100 mM d'un tampon phosphate (pH 7,0), à un débit de 30 ml/h. Les fractions (2 à 3 ml) sont collectées et l'hydroxamate dosé (absorbance $A_{526nm}$ --●--), sont également mesurées, l'inhibition de la croissance de Plasmodium falciparum (——o——) et l'absorbance $A_{220nm}$ (——△——).

. Inhibition de la croissance de Plasmodium falciparum :

0,2 µm des fractions filtrées sont introduites dans une culture de Plasmodium falciparum synchronisée, à une concentration finale de 2,5%, comme décrit dans le Tableau I; 370 kBg/ml (100 µCi/ml) de méthionine marquée au soufre 35 (Amersham) sont ajoutées au jour 1 (anneaux); la culture de parasite est collectée au jour 2 (schizontes) et l'incorporation de $^{35}$S-méthionine est mesurée dans du TCA à 10%.

La concentration en aérobactine du pool d'hydroxamate collecté après la filtration sur gel est estimée en comparaison avec le titre en hydroxamate d'une solution de L-Lysine hydroxamate à une concentration connue.

3. Mise en évidence de l'action anti-parasitaire

On a comparé l'action "antiplasmodiale" de trois chélateurs de fer in vitro vis-à-vis de Plasmodium falciparum et on a également étudié l'effet de l'addition de fer sur la culture de parasites (figure 4).

L'hydrochlorure de L-Lysine hydroxamate ne présente pas d'activité "antiplasmodiale", ceci indiquant que le groupe hydroxamate n'intervient pas seul dans l'activité "plasmocidine" de l'aérobactine.

La desferioxamine, un hydroxamate chélateur du fer utilisé pour provoquer une déprivation en fer dans les surcharges en fer, chez l'homme, inhibe la croissance des parasites.

L'inhibition par la desferioxamine est totalement réversée par l'addition de fer dans la culture de para sites.

Au contraire, l'addition du fer à des cultures de parasites traitées à l'aérobactine ne réverse pas l'action "plasmocidine" de l'aérobactine.

Ainsi la déprivation en fer joue un rôle dans l'activité "antiplasmodiale" de la desferioxamine mais pas dans l'activité "plasmocidine" de l'aérobactine. Ceci indique que l'aérobactine agit probablement comme une toxine et non par simple déprivation en fer.

La figure 4a montre l'inhibition de la croissance de Plasmodium falciparum par la L-Lysine hydroxamate (——▲——), la desferioxamine (——●——) et l'aérobactine (——o——), la mesure étant effectuée comme décrit à la figure 3.

L'addition de $2.10^{-3}$ M de $FeCl_3$ à la culture de parasites, immédiatement après l'hydroxamate a peu d'effet sur l'inhibition due à l'aérobactine (--o--) alors qu'elle réverse complètement l'inhibition due à la desferioxamine (——▲——).

La figure 4b montre le rôle de la concentration en $FeCl_3$ dans la réversion de l'inhibition due à la desferioxamine.

——●——pas de $FeCl_3$
——o——$2.10^{-4}$ M $FeCl_3$
——△——$10^{-3}$ M $FeCl_3$
——□——$2.10^{-3}$ M $FeCl_3$

4. Tests quantitatifs

Des tests in vitro ont montré notamment que l'aérobactine est capable d'inhiber à 100% la croissance de Plasmodium falciparum à une concentration de 2,5 µg/ml (environ $10^{-5}$ M).

Un autre essai réalisé in vivo a montré que l'injection intraveineuse (15 µg/souris) d'aérobactine purifiée à des souris infectées par Plasmodium chaubaudi entraîne un ralentissement de la multiplication des para-traitées avec 7,5 µg d'aérobactine, ou non traitées, ne survivent pas, l'injection d'aérobactine ne présente pas de toxicité pour la souris. Le même effet a été observé chez la souris infectée par P. falciparum comme le montre la figure 5 dans laquelle on a porté en abscisse le nombre de jours de survie et en ordonnée le pourcentage de ralentissement de la multiplication des parasites.

5. Spécificité de l'action anti-parasitaire

Un autre essai a montré l'action spécifique de l'aérobactine sur les cellules parasitées; en effet, l'aérobactine pénètre rapidement dans les globules rouges parasités et est concentrée au moins 200 fois dans la cellule parasitée. Elle n'est pas concentrée dans des globules rouges sains. La desferioxamine ne pénètre pas dans les globules rouges parasités.

Cet essai étudie l'incorporation de lysine marquée au tritium dans des globules rouges normaux et des globules rouges infectés par Plasmodium falciparum.

La figure 6 présente les résultats.

L'aérobactine marquée au tritium (7,4 à 11,1 MBg/mmol (0,2 à 0,3 mCi/mmol)) est purifiée comme décrit précédemment (figure 3) à partir d'un surnageant de culture de souches K5 1101 de Klebsiella pneumoniae, cultivée dans un milieu RPMI* contenant 3,7 MBg/ml (100 µCi/ml) de lysine tritiée.

La desferioxamine marquée à l'iode 125 (1850 MBg/mmol (50 mCi/mmol)) est obtenue par la technique iodogène.

L'aérobactine tritiée (7 µg/ml) et la desferioxamine $I^{125}$ (50 µg/ml) sont introduites dans des cultures synchronisées de Plasmodium falciparum (stade schizonte, 20% de parasitémie, hématocrite : 5%) et dans une suspension de globules rouges (5% hématocrite). Les cultures sont incubées à 37°C et des échantillons de 50 µl sont prélevés à des moments différents.

Pour chaque échantillon, on mesure :

1. les cpm dans le surnageant de culture,
2. les cpm dans les surnageants de la lyse des globules rouges (les globules rouges sont lysés dans 40 vol. de RPMI dix fois dilué et les parasites libres sont précipités à l'aide d'une centrifugation 5000 rpm pendant 5 min et lavés dans du RPMI),
3. les cpm associés aux parasites libres.

Les parasites sont dans un deuxième temps lysés pour préparer les fractions solubles et membranaires comme décrit précédemment (BRAUN-BRETON et al., Molecular Biochem. Parasitol., 1986, 20, 33-43).

La radioactivité est essentiellement retrouvée dans la fraction soluble car l'aérobactine intraparasitaire est récupérée dans la fraction soluble des parasites.

L'aérobactine n'est pas détectée dans le cytoplasme des globules rouges.

——△——: aérobactine extracellulaire
——●——: aérobactine associée aux globules rouges
——o—— : aérobactine associée aux parasites
——□——: desferioxamine extracellulaire
——■——: desferioxamine intracellulaire

## 6. Etude de la toxicité des produits selon l'invention

Un autre essai sur la toxicité des produits selon l'invention montre qu'aucun effet toxique après l'injection d'aérobactine n'a été observé chez les souris ou les singes.

Par ailleurs, l'addition d'aérobactine purifiée à des fibroblastes en culture n'a aucun effet toxique sur ces cellules.

## 7. Cinétique d'action sur les parasites

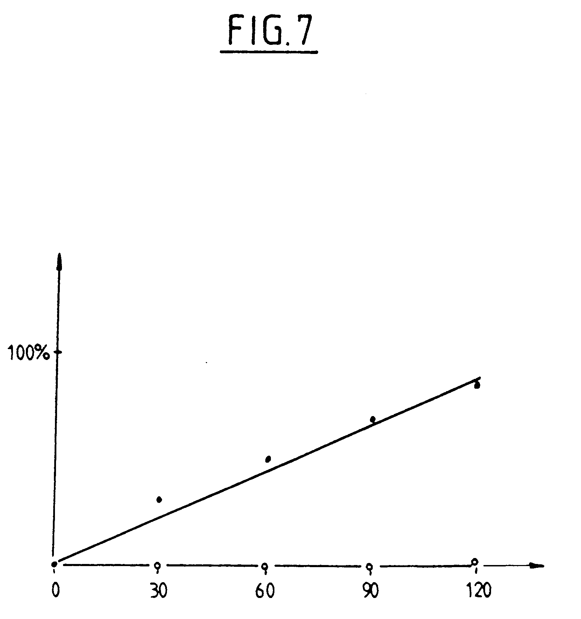

Comme il est indiqué sur la figure 7, l'aérobactine réprime rapidement l'incorporation de $S^{35}$-méthionine dans les protéines des schizontes.

En abscisse on a le temps en minutes et en ordonnée le pourcentage de schizontes inhibés.

Les conditions opératoires sont les suivantes :

– Les schizontes de Plasmodium falciparum sont concentrés à partir d'une culture, lavés et cultivés à 37°C pendant une heure. Des solutions d'hydroxamate ( ——●—— 50 µg/ml d'aérobactine et ——o—— 500 µg/ml de desferioxamine) sont ajoutés aux schizontes. L'incubation à 37°C est poursuivie et des échantillons sont prélevés à des temps différents; l'hydroxamate est retiré par lavage des cellules et celles-ci sont à nouveau cultivées en présence de 9,25 MBg (250 µCi/ml) $S^{35}$-méthionine pendant une heure.

– Les cultures sont récoltées et l'incorporation de la $S^{35}$-méthionine est mesurée.

– On voit que l'aérobactine inhibe l'incorporation de méthionine chez 90% des schizontes en 2 heures alors que la desferioxamine n'a aucune action sur l'incorporation de méthionine.

Une telle activité n'a pas été retrouvée lorsque l'aérobactine est introduite dans des cultures de fibroblastes L pendant 5 heures.

## Revendications

### Revendications pour les Etats contractants suivants: AT, BE, CH, DE, FR, GB, IT, LI, LU, NL, SE

1. Utilisation d'une substance constituée par un produit sidérophore comprenant au moins un groupe hydroxamate, au moins une liaison peptidique, et un poids moléculaire inférieur à 1000 D, et présentant la propriété de pénétrer dans les cellules, notamment dans les globules rouges, pour l'obtention d'un médicament destiné à une utilisation anti-parasitaire, notamment antimalarique, spécifique et non réversée par le fer, vis-à-vis des parasites endocellulaires.

2. Utilisation d'une substance telle que définie à la revendication 1, caractérisée en ce qu'il s'agit de l'aérobactine de formule I ci-après :

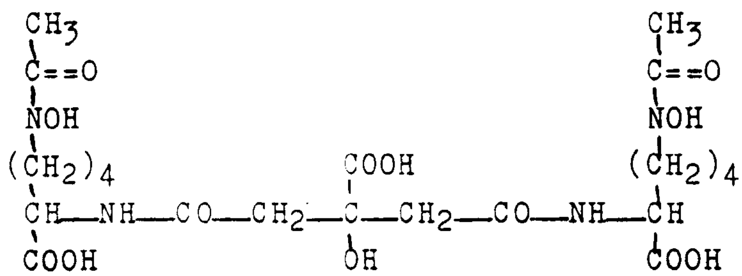

(I)

laquelle formule englobe également les produits dérivés par substitution.

3. Compositions pharmaceutiques, caractérisées en ce qu'elles comprennent une quantité active d'un produit anti-parasitaire sidérophose comprenant au moins un groupe hydroxamate, au moins une liaison peptidique, et un poids moléculaire inférieur à 1000 D, et présentant la propriété de pénétrer dans les cellules, notamment dans les globules rouges, éventuellement associé à un véhicule d'administration approprié.

4. Compositions pharmaceutiques selon la revendication 3, caractérisées en ce que lorsqu'elles sont présentées sous forme retard, le/les principe(s) actif(s) est/sont adsorbés sur du phosphate de calcium.

### Revendications pour les Etats contractants suivants: ES, GR

1. Application d'une substance constituée par un produit sidérophore comprenant au moins un groupe hydroxamate, au moins une liaison peptidique, et un poids moléculaire inférieur à 1000 D, et présentant la propriété de pénétrer dans les cellules, notamment dans les globules rouges, pour la préparation d'un médicament destiné à une utilisation anti-parasitaire, notamment antimalarique, spécifique et non réversée par le fer, vis-à-vis des parasites endocellulaires.

2. Application d'une substance telle que définie à la revendication 1, caractérisée en ce qu'il s'agit de l'aérobactine de formule I ci-après :

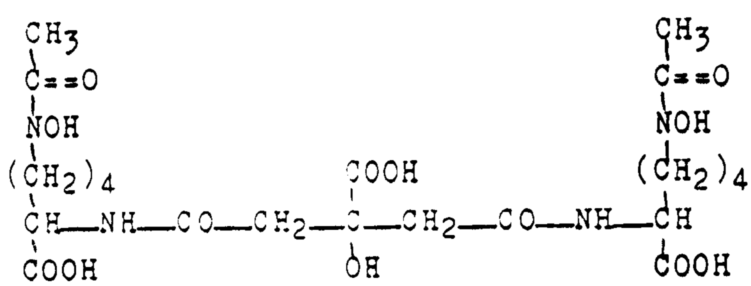

(I)

laquelle formule englobe également les produits dérivés par substitution.

3. Procédé de préparation de compositions pharmaceutiques, caractérisé en ce qu'il met en oeuvre une quantité active d'un produit anti-parasitaire sidérophore comprenant au moins un groupe hydroxamate, au

moins une liaison peptidique, et un poids moléculaire inférieur à 1000 D, et présentant la propriété de pénétrer dans les cellules, notamment dans les globules rouges, éventuellement associé à un véhicule d'administration approprié.

4. Procédé selon la revendication 3, caractérisé en ce que lesdites compositions lorsqu'elles sont présentées sous forme retard, le/les principe(s) est/sont adsorbés sur du phosphate de calcium.

**Patentansprüche**

**Patentansprüche für folgende Vertragsstaaten: AT, BE, CH, DE, FR, GB, IT, LI, LU, NL, SE**

1. Verwendung einer Substanz gebildet aus einem siderophoren Produkt, welches wenigstens eine Hydroxamatagruppe, wenigstens eine Peptidbindung und ein Molekulargewicht von weniger als 1000 D aufweist und die Eigenschaft besitzt, in die Zellen, insbesondere in die roten Blutkörperchen, einzudringen, für die Herstellung eines Medikaments, welches für eine spezifische und durch Eisen nicht reversierte antiparasitäre Anwendung, insbesondere Antimalariaanwendung, gegenüber endozelluläre Parasiten bestimmt ist.

2. Verwendung einer wie in Anspruch 1 definierten Substanz dadurch gekennzeichnet, daß es sich um ein Aerobactin der nachstehenden Formel I:

```
 CH3                                     CH3
 |                                       |
 C==O                                    C==O
 |                                       |
 NOH                                     NOH
 |                                       |
(CH2)4                  COOH            (CH2)4
 |                      |                |
 CH__NH__CO__CH2__C__CH2__CO__NH__CH
 |                      |                |
 COOH                   OH               COOH

                                                  (I)
```

welche Formel zugleich die durch Substitution abgeleiteten Produkte umfaßt, handelt.

3. Pharmazeutische Zusammensetzungen, dadurch gekennzeichnet, daß sie eine aktive Menge eines antiparasitären siderophoren Produkts, das wenigsten eine Hydroxamatgruppe, wenigstens eine Peptidbindung und ein Molekulargewicht von weniger als 1000 D aufweist und die Eigenschaft besitzt, in die Zellen, insbesondere in die roten Blutkörperchen, einzudringen, gegebenenfalls in Verbindung mit einem geeigneten Verabreichungsträger enthalten.

4. Pharmazeutische Zusammensetzung nach Anspruch 3, dadurch gekennzeichnet, daß, falls sie in Retardform angeboten werden, der aktive Bestandteil/die aktiven Bestandteile auf Kalziumphosphat adsorbiert ist/sind.

**Patentansprüche für folgende Vertragsstaaten: ES, GR**

1. Verwendung einer Substanz gebildet aus einem siderophoren Produkt, welches wenigestens eine Hydroxamatagruppe, wenigstens eine Peptidbindung und ein Molekulargewicht von weniger als 1000 D aufweist und die Eigenschaft besitzt, in die Zellen, insbesondere in die roten Blutkörperchen, einzudringen, für die Herstellung eines Medikaments, welcher für eine spezifische und durch Eisen nicht reversierte antiparasitäre Anwendung, insbesondere Antimalariaanwendung, gegenüber endozelluläre Parasiten bestimmt ist.

2. Verwendung einer wir in Anspruch 1 definierten Substanz dadurch gekennzeichnet daß es sich um ein

Aerobactin der nachstehenden Formel I:

```
CH3                                         CH3
|                                           |
C==O                                        C==O
|                                           |
NOH                                         NOH
|                                           |
(CH2)4                  COOH                (CH2)4
|                       |                   |
CH__NH__CO__CH2__C__CH2__CO__NH__CH
|                       |                   |
COOH                    OH                  COOH

                                                        (I)
```

welche Formel zugleich die durch Substitution abgeleiteten Produkte umfaßt, handelt.

3. Verfahren zur Herstellung von pharmazeutischen Zusammensetzungen, dadurch gekennzeichnet, daß man eine aktive Menge eines antiparasitären siderophoren Produkts, das wenigestens eine Hydroxamatgruppe, wenigstens eine Peptidbindung und ein Molekulargewicht von weniger als 1000 D aufweist und die Eigenschaft besitzt, in die Zellen, insbesondere in die roten Blutkörperchen, einzudringen, gegebenenfalls in Verbindung mit einem geeigneten Verabreichungsträger einsetzt.

4. Verfahren nach Anspruch 3, dadurch gekennzeichnet, daß falls die genannten Zusammensetzungen in Retardform angeboten werden, der Bestandteil/die Bestandteile auf Kalziumphosphat adsorbiert wird/werden.

## Claims

### Claims for the following Contracting States: AT, BE, CH, DE, FR, GB, IT, LI, LU, NL, SE

1. Use of a substance consisting of a siderophoric product comprising at least one hydroxamate group, at least one peptide bond, and a molecular weight lower than 1000 D, and exhibiting the property of penetrating into the cells, especially in red blood cells, for the obtaining of a drug designed for anti-parasitic use, especially anti-malarial, specific and not reversed by iron, with respect to endocellular parasites.

2. Use of a substance as defined in Claim 1, characterized in that it is aerobactin with the formula (I) below:

```
CH3                                  CH3
|                                    |
C==O                                 C==O
|                                    |
NOH                                  NOH
|                                    |
(CH2)4              COOH             (CH2)4
|                   |                |
CH—NH—CO—CH2—C—CH2—CO—NH—CH
|                   |                |
COOH                OH               COOH                (I)
```

the formula also including products derived by substitution.

3. Pharmaceutical compositions, characterized in that they comprise an active quantity of an anti-parasitic,

siderophoric product comprising at least one hydroxamate group, at least one peptide bond, and a molecular weight lower than 1000 D, and exhibiting the property of penetrating into the cells, especially in red blood cells, possibly combined with an appropriate vehicle for administration.

4. Pharmaceutical compositions according to Claim 3, characterized in that when they are presented in delayed form, the active ingredient(s) is/are adsorbed on calcium phosphate.

**Claims for the following Contracting States: ES, GR**

1. Application of a substance consisting of a siderophoric product comprising at least one hydroxamate group, at least one peptide bond, and a molecular weight lower than 1000 D, and exhibiting the property of penetrating into the cells, especially in red blood cells, for the obtaining of a drug designed for anti-parasitic use, especially anti-malarial, specific and not reversed by iron, with respect to endocellular parasites.

2. Application of a substance as defined in Claim 1, characterized in that it is aerobactin with the formula (I) below:

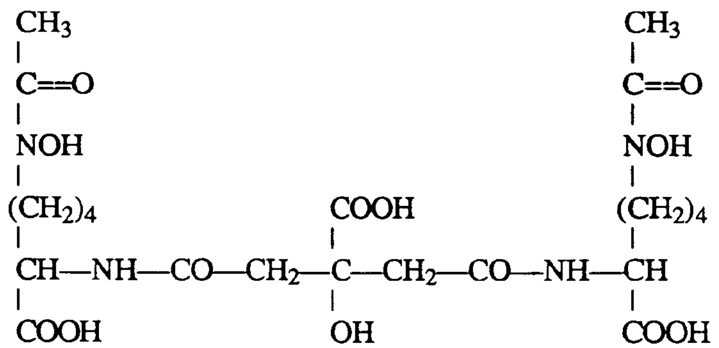

(I)

the formula also including products derived by substitution.

3. Process for the preparation of pharmaceutical compositions, characterized in that it employs an active quantity of an anti-parasitic siderophoric product comprising at least one hydroxamate group, at least one peptide bond, and a molecular weight lower than 1000 D, and exhibiting the property of penetrating into the cells, especially into red blood cells, possibly combined with an appropriate vehicle for administration.

4. Process according to Claim 3, characterized in that the said compositions, when presented in delayed form, the active ingredient(s) is/are adsorbed on calcium phosphate.

FIG. 1

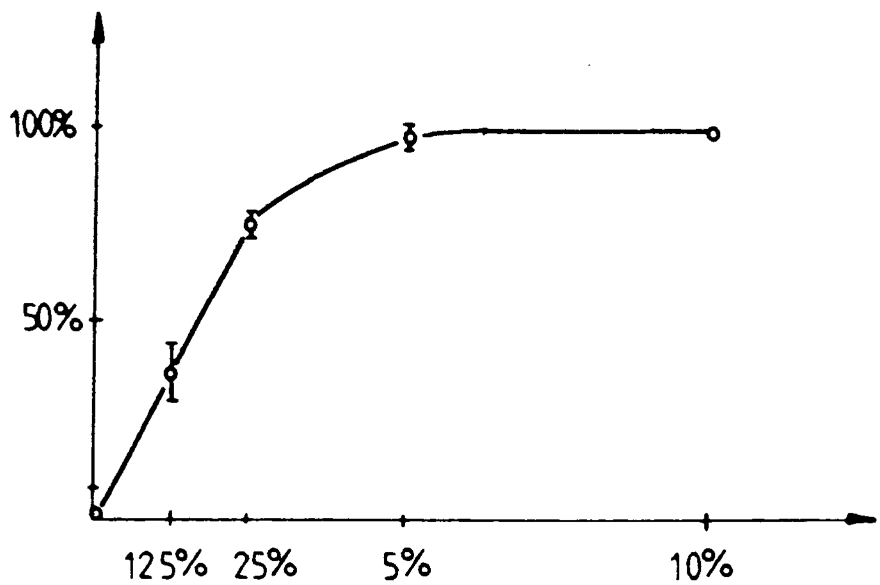

FIG. 2

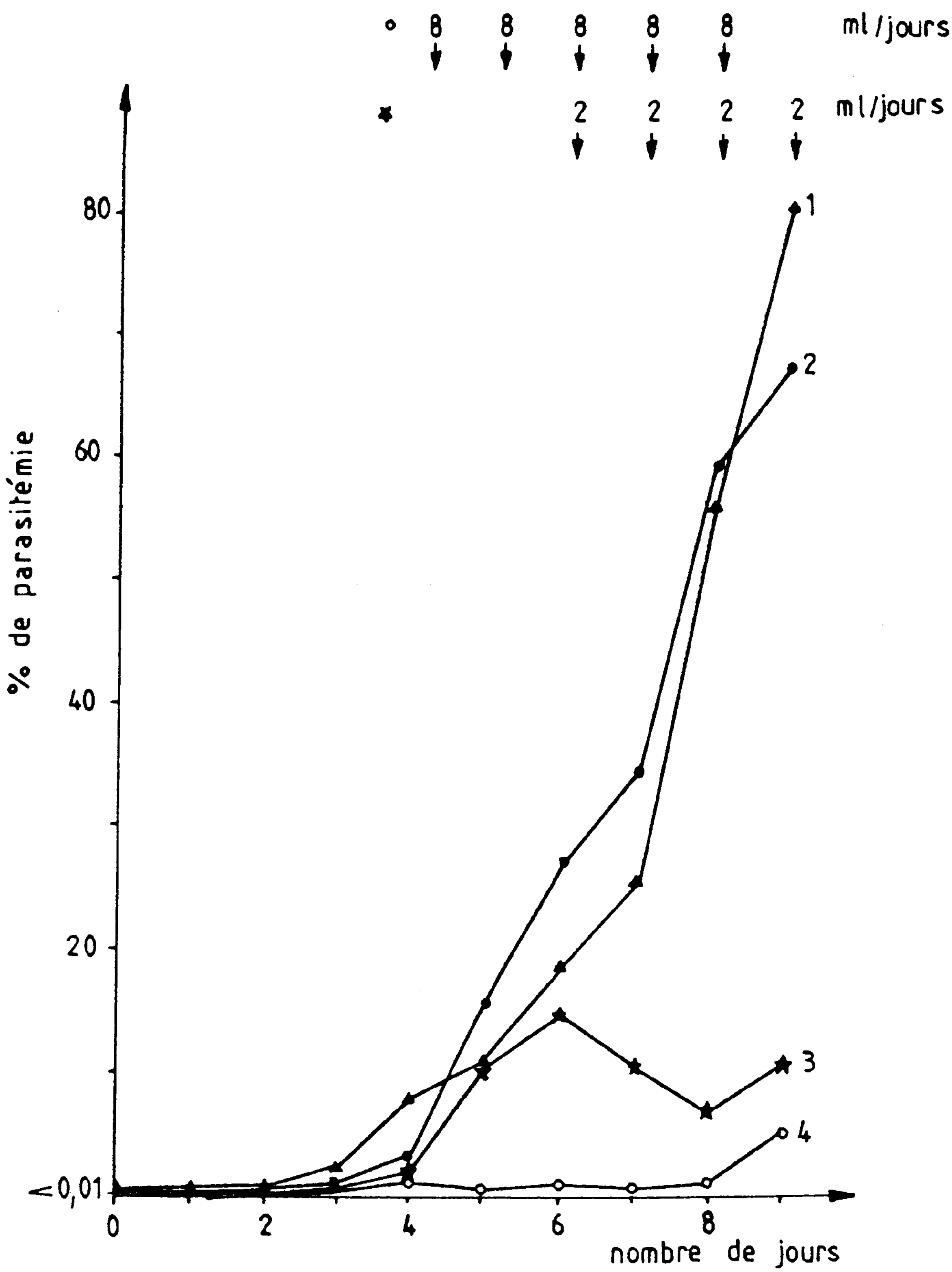
○ 8 8 8 8 8 ml/jours
✱ 2 2 2 2 ml/jours
80
60
40
20
<0,01
% de parasitémie
0
2
4
6
8
nombre de jours
1
2
3
4

FIG. 3

a)

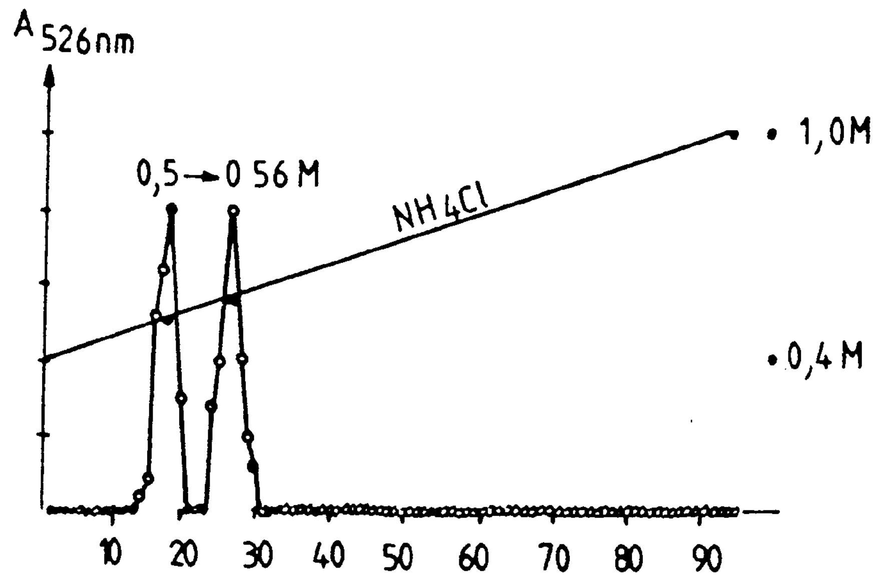
A 526nm
0,5 → 0 56 M
NH4Cl
1,0M
0,4 M
10
20
30
40
50
60
70
80
90

b)

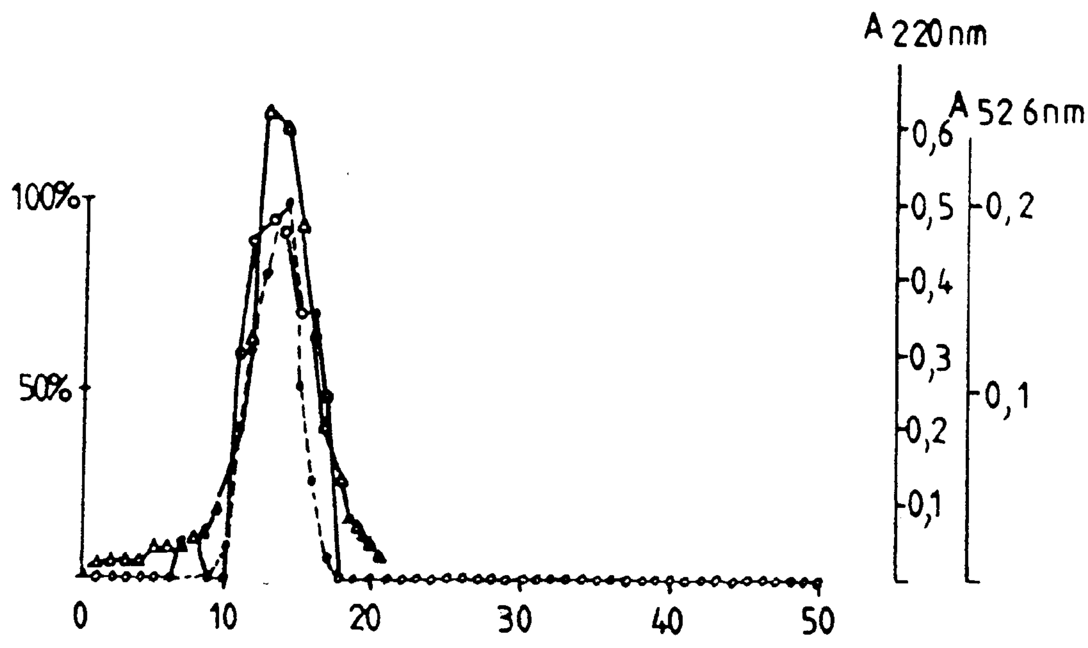
A 220nm
A 526nm
100%
50%
0,6
0,5
0,4
0,3
0,2
0,1
0,2
0,1
0
10
20
30
40
50

FIG. 4

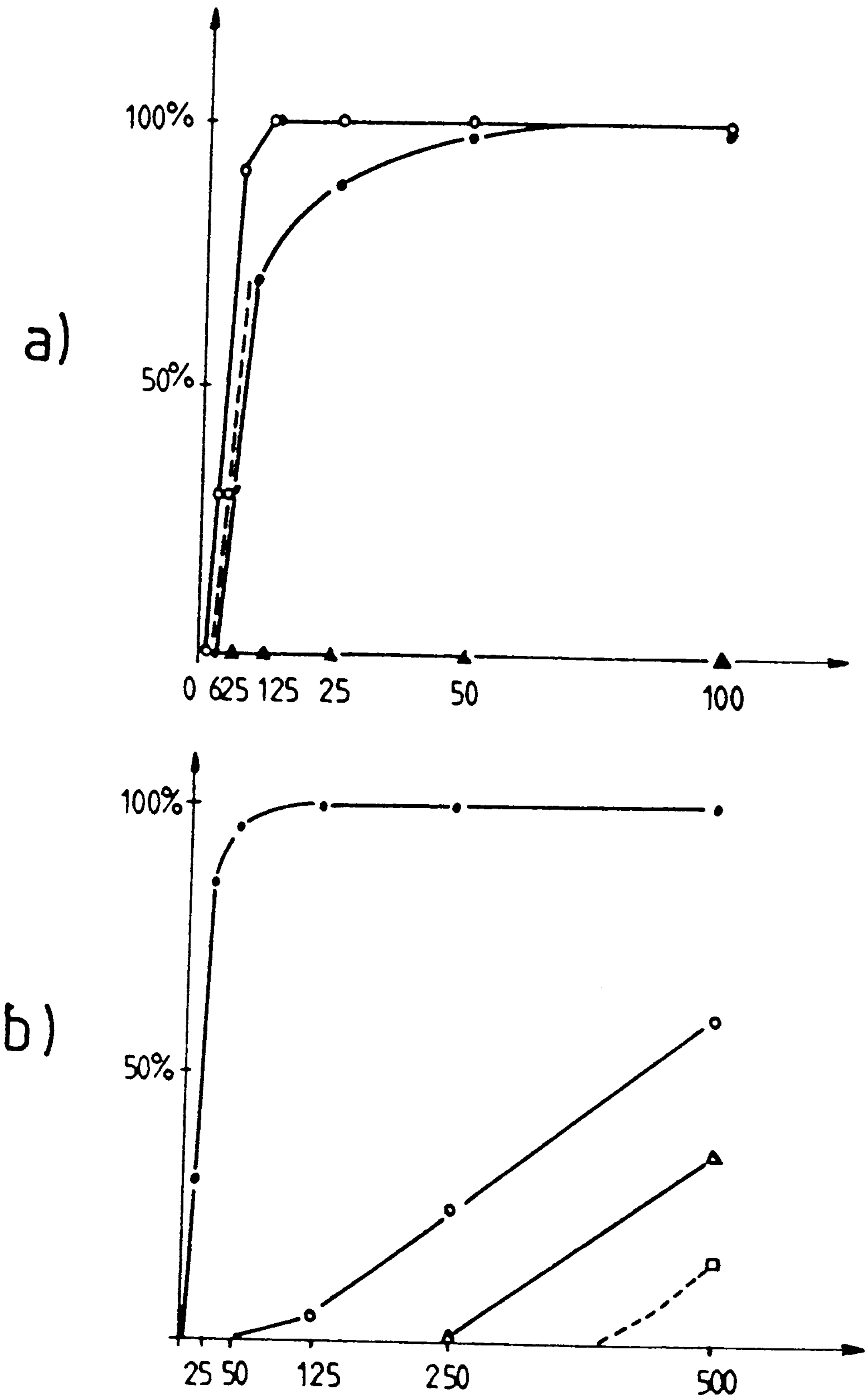

FIG.5

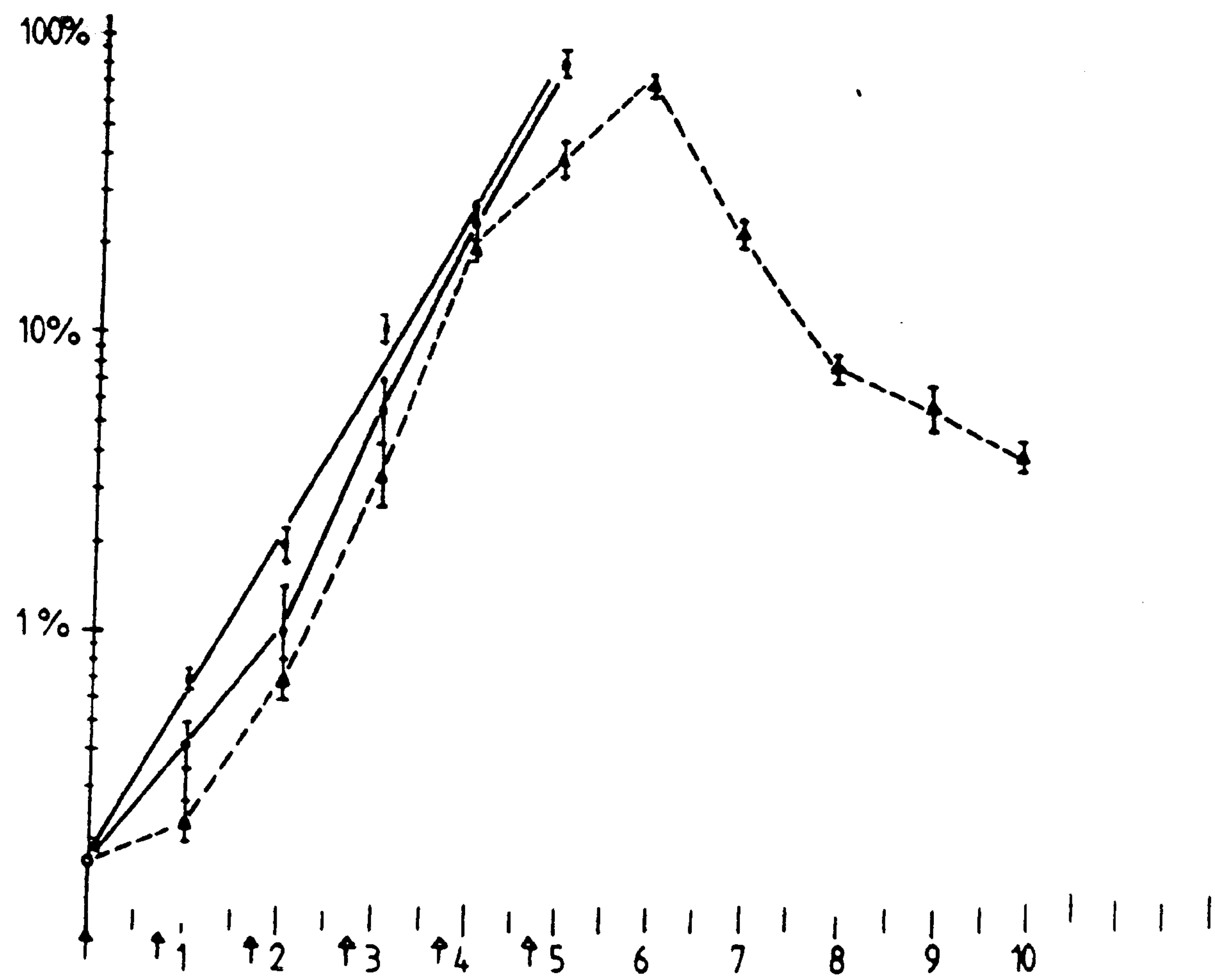

FIG. 6

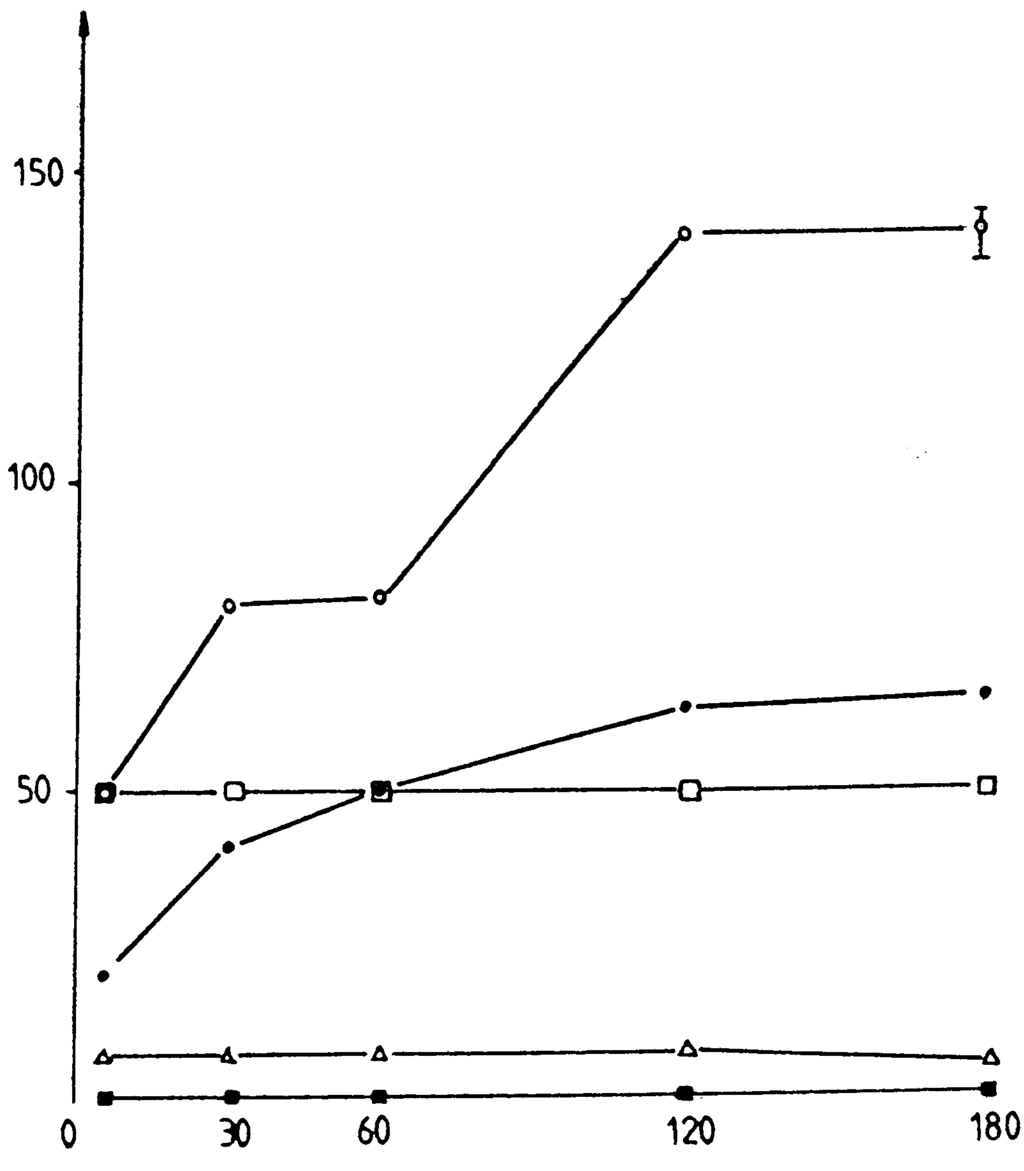

FIG. 7